(19) Europäisches Patentamt

European Patent Office

Office européen des brevets

(11) **EP 1 077 083 B1**

(12) **FASCICULE DE BREVET EUROPEEN**

(45) Date de publication et mention
de la délivrance du brevet:
**21.06.2006 Bulletin 2006/25**

(51) Int Cl.:
*B01J 29/04* *(2006.01)*    *C01B 39/02* *(2006.01)*
*C07C 6/12* *(2006.01)*    *B01J 29/78* *(2006.01)*

(21) Numéro de dépôt: **00402217.4**

(22) Date de dépôt: **03.08.2000**

(54) **Catalyseur comprenant au moins une zéolite de type structural NES et du rhénium et son utilisation en transalkylation d'hydrocarbures alkylaromatiques**

Katalysator, zumindest eine Zeolite des NES-Typs enthaltend, und Rhenium und seiner Verwendung für die Transalkylierung von aromatischen Kohlenwasserstoffen

Catalyst comprising at least one zeolite of the NES structural type and rhenium and the transalkylation of aromatic hydrocarbons using the same

(84) Etats contractants désignés:
**BE DE ES GB IT NL**

(30) Priorité: **19.08.1999  FR 9910652**

(43) Date de publication de la demande:
**21.02.2001  Bulletin 2001/08**

(73) Titulaire: **Institut Français du Pétrole**
**92852 Rueil-Malmaison Cedex (FR)**

(72) Inventeurs:
• **Merlen, Elisabeth**
**92500 Rueil-Malmaison (FR)**

• **Alario, Fabio**
**92200 Neuilly sur Seine (FR)**
• **Ferrer, Nathalie**
**78360 Montesson (FR)**
• **Martin, Olivia**
**92000 Nanterre (FR)**

(56) Documents cités:
**EP-A- 0 825 151        EP-A- 0 925 822**
**FR-A- 2 765 237        US-A- 5 254 787**

Remarques:
Le dossier contient des informations techniques présentées postérieurement au dépôt de la demande et ne figurant pas dans le présent fascicule.

EP 1 077 083 B1

**Description**

Domaine technique

La présente invention concerne un catalyseur utilisable par exemple dans les réactions de transformation des hydrocarbures aromatiques. De manière plus précise, elle concerne un catalyseur de transalkylation d'hydrocarbures alkylaromatiques et de préférence de transalkylation du toluène et de composés aromatiques contenant au moins 9 atomes de carbone, pour produire des xylènes. La présente invention concerne aussi la préparation dudit catalyseur ainsi que son utilisation dans un procédé de transalkylation d'hydrocarbures alkylaromatiques.

Art antérieur

De nombreux catalyseurs de dismutation et/ou de transalkylation ont déjà été décrits dans l'art antérieur, et sont à base de mordénite (US-A-3.506.731, US-A-4.151.120, US-A-4.180.693, US-A-4.210.770, US-A-3.281.483, US-A-3.780.121, ou US-A-3.629.351), ou bien aussi à base de zéolithe oméga (US-A-5.210.356, US-A-5.371.311).

Il a été décrit dans le brevet EP-B1-0.378.916, la zéolithe NU-87, qui est une zéolithe de type structural NES, ainsi que son mode de préparation en présence d'un sel d'un cation polyméthylène diammonium par exemple le bromure de décaméthonium. Dans ce brevet, le rhénium est cité parmi de nombreux autres éléments pour ses propriétés hydrodéshydogénantes.

Le brevet US-A-5,641,393 traite de la zéolithe SSZ-37, dont le rapport $SiO_2/Al_2O_3$ de la zéolithe brute de synthèse est supérieur à 400. La synthèse de cette zéolithe est différente de celle de la NU-87 en ce que le structurant est le cation N,N,diméthyl-4-azoniatricyclo[5.2.2.0$^{(2,6)}$]undec-8-ène pour la zéolithe SSZ-37.

L'intérêt de la zéolithe NU-87, de type structural NES, en dismutation et/ou transalkylation d'hydrocarbures alkylaromatiques a été montré dans le brevet FR 2.752.568 de la demanderesse. Dans ce brevet, l'intérêt de l'ajout d'un métal comme le nickel est également abordé.

Les documents EP-A1-0.325.822, FR-A1-2.765.237, EP-A1-0.825.151, EP-A1-0.938.926 et EP-A1-0.980.708 divulguent des catalyseurs à base de zéolithe NU-87.

Dans le brevet EP-A1-0.731.071, l'utilisation d'un catalyseur à base de zéolithe mordénite et de rhénium est décrit en transalkylation de coupes C9 aromatiques contenant un aromatique ayant au moins un groupement éthyle. Bien que le rhénium soit le métal préféré, d'autres métaux (Ni, Co, Mo, Cr et W) sont cités comme convenant à l'invention.

Résumé

Le catalyseur de la présente invention contient au moins une zéolithe de type structural NES, de préférence la NU-87, comprenant du silicium et au moins un élément T choisi dans le groupe formé par l'aluminium, le fer, le gallium, le bore. l'élément T a été extrait de façon à ce que le rapport Si/T atomique global soit supérieur à 20. Cette zéolithe est présente au moins en partie sous forme acide. Le liant est de préférence l'alumine. Le catalyseur contient en outre au moins le rhénium. Le catalyseur contient, en plus, au moins un métal choisi dans le groupe formé par les éléments des groupes IIIA et IVA de la Classification périodique des Eléments, de préférence l'indium et l'étain. La présente invention concerne également l'utilisation du catalyseur dans un procédé de transalkylation d'hydrocarbures alkylaromatiques tels que le toluène et les alkylaromatiques ayant au moins 9 atomes de carbone. Ce catalyseur est, en particulier, très performant dans le traitement de charges aromatiques C9+ contenant un pourcentage de molécules aromatiques ayant au moins 10 atomes de carbone et plus supérieur à 5 % poids, cette charge pouvant également contenir du benzène.

Intérêt

Il a été découvert, qu'un catalyseur contenant au moins une zéolithe de type structural NES, de préférence la zéolithe NU-87 ayant en rapport Si/T supérieur à environ 20 de l'élément T ayant été extrait ds la zéolithe au moins en partie, de préférence pratiquement totalement, sous forme acide, au moins du rhénium, et au mans un métal choisi dans le groupe forme par les métaux des groupes IIIA et IVA conduit à des performances catalytiques, et en particulier à des activités, stabilités et sélectivités, améliorées dans les réactions de transalkylations d'hydrocarbures alkylaromatiques tels que le toluène et les alkylaromatiques à au moins 9 atomes de carbone par rapport aux catalyseurs de l'art antérieur. Ce catalyseur est, en particulier, très performant dans le traitement de charges aromatiques C9+ contenant un pourcentage élevé de molécules aromatiques à 10 atomes de carbone et plus (supérieur à 5 % poids) ce qui permet de valoriser ces molécules lourdes (comme par exemple les diméthyléthylbenzènes, les diéthylbenzènes ...) en xylènes, tout en présentant des sélectivités ainsi qu'une stabilité améliorées par rapport aux catalyseurs de l'art antérieur, ce qui permet en outre de produire du benzène de pureté améliorée.

Description

L'invention concerne donc un catalyseur contenant au moins une zéolithe de type structural NES, de préférence une zéolithe NU-87 à raison de 30 à 90 % et de préférence de 60 à 85 % poids et au moins une matrice (ou liant) en complément à 100 % du catalyseur. Ladite zéolithe, de préférence la NU-87, comprenant du silicium et au moins un élément T choisi dans le groupe formé par l'aluminium, le fer, le gallium, et le bore, de préférence l'aluminium, est, au moins en partie, de préférence pratiquement totalement, sous forme acide. De l'élément a été extrait de la zéolithe pour que le rapport Si/T atomique global de ladite zéolithe, soit supérieur à 20, de préférence supérieur à 25, et de manière préférée compris entre environ 25 et environ 350, ou de manière encore plus préférée entre 25 et 250, ou encore entre environ 25 et environ 150. Lorsqu'elle est comprise dans le catalyseur selon l'invention, la zéolithe de type structural NES est au moins en partie, de préférence pratiquement totalement, sous forme acide, c'est-à-dire sous forme hydrogène ($H^+$). La teneur en sodium est inférieure à 0,1 % poids, de préférence inférieure à 0,05 % poids par rapport au poids total de zéolithe sèche.

Ledit catalyseur comprend en outre au moins du rhénium, à une teneur comprise entre 0,01. et 5 % et de préférence entre 0,05 et 3 % poids, et au moins un élément choisi dans l'ensemble formé par les groupes IIIA et IVA de la classification périodique des éléments, de préférence choisi dans le groupe formé par l'indium et l'étain à une teneur comprise entre 0,01 et 5 % et de préférence entre 0,5 et 3 % poids.

La matrice, présente à une teneur comprise entre 10 et 60 % et de préférence entre 15 et 40 % poids par rapport au poids total de catalyseur, est choisie généralement parmi les éléments du groupe formé par les argiles (par exemple parmi les argiles naturelles telles que le kaolin ou la bentonite), la magnésie, les alumines, les silices, l'oxyde de titane, l'oxyde de bore, la zircone, les phosphates d'aluminium, les phosphates de titane, les phosphates de zirconium, les silice-alumines et le charbon, de préférence parmi les éléments du groupe formé par les alumines et les argiles, de manière encore plus préférée parmi les alumines.

La présente invention concerne également la préparation du catalyseur.

La zéolithe NES comprise dans le catalyseur selon la présente invention est de préférence la zéolithe NU-87 préparée selon le brevet EP 0 378 916 B1. Ainsi, la préparation de la zéolithe NU-87 comprend le mélange d'une source de silicium et d'une source d'un élément T, un cation alcalin et un structurant organique choisi parmi les sels de cations de polyméthylène diammonium, par exemple le bromure de décaméthonium.

La zéolithe NES utilisée dans le catalyseur de la présente invention est telle que de l'élément T a été extrait de la charpente.

Pour préparer la zéolithe désaluminée de type structural NES dans le cas préféré où l'élément T est l'aluminium, deux méthodes de désalumination peuvent être employées, à partir de la zéolithe de type structural NES brute de synthèse comprenant du structurant organique. Elles sont décrites ci-après. Mais toute autre méthode connue de l'homme du métier entre aussi dans le cadre de l'invention.

Ces méthodes décrites pour l'aluminium peuvent également être valables pour les autres éléments T.

La première méthode dite de l'attaque acide directe comprend une première étape de calcination sous flux d'air sec, à une température généralement comprise entre environ 450 et 550°C, qui a pour but d'éliminer le structurant organique présent dans la microporosité de la zéolithe, suivie d'une étape de traitement par une solution aqueuse d'un acide minéral tel que $HNO_3$ ou HCl ou organique tel que $CH_3CO_2H$. Cette dernière étape peut être répétée autant de fois qu'il est nécessaire afin d'obtenir le niveau de désalumination voulu. Entre ces deux étapes (calcination sous air sec et attaque acide directe) il est possible de réaliser un ou plusieurs échanges ioniques par au moins une solution $NH_4NO_3$, de manière à éliminer au moins en partie, de préférence pratiquement totalement, le cation alcalin, en particulier le sodium. De même, à la fin du traitement de désalumination par attaque acide directe, il est éventuellement possible de réaliser un ou plusieurs échanges ioniques par au moins une solution $NH_4NO_3$, de manière à éliminer les cations alcalins résiduels et en particulier le sodium.

Pour atteindre le rapport Si/Al désiré, il est nécessaire de bien choisir les conditions opératoires ; de ce point de vue les paramètres les plus critiques sont la température de traitement par la solution aqueuse d'acide, la concentration dudit acide, la nature dudit acide, le rapport entre la quantité de solution acide et la masse de zéolithe traitée, la durée de traitement et le nombre de traitements réalisés.

On peut également effectuer des traitements de désalumination par des composés chimiques désaluminants tels que (à titre d'exemples et de façon non exhaustive) le tétrachlorure de silicium ($SiCl_4$), l'hexafluorosilicate d'ammonium [$(NH4)_2SiF_6$], l'acide éthylènediaminetétracétique (EDTA) ainsi que sa forme mono et disodique. Ces réactifs peuvent être utilisés en solution ou en phase gaz par exemple dans le cas de $SiCl_4$.

La deuxième méthode de désalumination dite de traitement thermique (en particulier à la vapeur d'eau ou "steaming") suivi d'une attaque acide, comprend, dans un premier temps, la calcination sous flux d'air sec, à une température généralement comprise entre environ 450 et 550°C, qui a pour but d'éliminer le structurant organique occlus dans la microporosité de la zéolithe. Puis le solide ainsi obtenu est soumis à un ou plusieurs échanges ioniques par au moins une solution $NH_4NO_3$, de manière à éliminer au moins en partie, de préférence pratiquement totalement, le cation alcalin, en particulier le sodium, présent en position cationique dans la zéolithe. La zéolithe ainsi obtenue est soumise à au moins un cycle de désalumination de charpente, comportant au moins un traitement thermique réalisé, éventuellement et de préférence en présence de vapeur d'eau, à une température généralement comprise entre 550 et 900°C, et éventuellement suivie d'au moins une attaque acide par une solution aqueuse d'un acide minéral ou organique. Les conditions de calcination en présence de vapeur d'eau (température, pression de vapeur d'eau et durée du traitement) ainsi que les conditions d'attaque acide post-calcination (durée de l'attaque, concentration de l'acide, nature de l'acide utilisé et rapport entre le volume d'acide et la masse de zéolithe), sont adaptées de manière à obtenir le niveau de désalumination souhaité. Dans le même but on peut aussi jouer sur le nombre de cycles traitement thermique - attaque acide qui sont effectués.

Une variante de cette deuxième méthode peut constituer à remplacer l'étape dite de l'attaque acide, c'est-à-dire du traitement par une solution d'acide, par un traitement par une solution d'un composé chimique désaluminant tels que, par exemple, ceux cités précédemment à savoir le tétrachlorure de silicium ($SiCl_4$), l'hexafluorosilicate d'ammonium [$(NH4)_2SiF_6$], l'acide éthylènediaminetétracétique (EDTA) ainsi que sa forme mono et disodique.

Dans le cas préféré où l'élément T est l'aluminium, le cycle de désalumination de la charpente, comportant au moins une étape de traitement thermique réalisé, éventuellement et de préférence, en présence de vapeur d'eau et au moins une étape d'attaque en milieu acide de la zéolithe de type structural NES, peut être répété autant de fois qu'il est nécessaire pour obtenir la zéolithe de type structural NES désaluminée possédant les caractéristiques désirées. De même, suite au traitement thermique réalisé, éventuellement et de préférence en présence de vapeur d'eau, plusieurs attaques acides successives, avec des solutions en acide de concentrations différentes, peuvent être opérées.

Une variante de cette deuxième méthode de désalumination comprend le traitement thermique de la zéolithe de type structural NES contenant le structurant organique, à une température généralement comprise entre 550°C et 900°C, éventuellement et de préférence en présence de vapeur d'eau. Dans ce cas, les étapes de calcination du structurant organique et de désalumination de la charpente par traitement thermique sont réalisées simultanément. Puis, la zéolithe est éventuellement traitée par au moins une solution aqueuse d'un acide minéral (par exemple de $HNO_3$ ou de HCl) ou organique ($CH_3CO_2H$ par exemple). Enfin, le solide ainsi obtenu peut être éventuellement soumis à au moins un échange ionique par au moins une solution $NH_4NO_3$, de manière à éliminer pratiquement tout cation alcalin, en particulier le sodium, présent en position cationique dans la zéolithe.

Dans une mise en oeuvre préférée de l'invention, on utilisera une méthode de désalumination conduisant à une diminution du nombre d'atomes d'aluminium dans la majeure partie du grain de la zéolithe et non pas uniquement à l'extrême surface des grains. De manière préférée, les zéolithes NES dans lesquelles de l'élément T, de préférence de l'aluminium a été extrait comprennent un réseau mésoporeux dans le grain de zéolithe qui peut être visualisé par microscopie électronique à transmission.

La préparation du catalyseur peut être poursuivie selon toute méthode connue de l'homme du métier. En général, elle est obtenue par mélange de la matrice et de la zéolithe puis par mise en forme. Le rhénium peut être introduit soit avant la mise en forme, ou bien lors du mélange, ou bien, et de préférence, après la mise en forme. On comprend ainsi que le mélange matrice + zéolithe est un support pour le catalyseur contenant le rhénium La mise en forme est généralement suivie d'une calcination, généralement à une température comprise entre 250 et 600 °C. Le rhénium peut être introduit après ladite calcination. Dans tous les cas, le phénium généralement déposé au choix soit pratiquement totalement sur la zéolithe, soit pratiquement totalement sur la matrice, soit en partie sur la zéolithe et en partie sur la matrice, ce choix s'effectuant de la manière qui est connue de l'homme du métier, par les paramètres utilisés lors dudit dépôt, comme par exemple la nature du précurseur choisi pour effectuer ledit dépôt.

Le rhénium, peut donc être déposé sur le mélange zéolithe-matrice préalablement mis en forme par tout procédé connu de l'homme de l'art. Un tel dépôt est généralement effectué par la technique d'imprégnation à sec, d'échange(s) ionique(s) ou de coprécipitation. Parmi les précurseurs, on peut citer de manière non limitative, l'acide perrhénique et le perrhénate d'ammonium, déposés, par exemple par imprégnation à sec.

Le dépôt de rhénium est suivi en général d'une calcination sous air ou oxygène, généralement entre 300 et 600°C, de préférence entre 350 et 550°C, et pour une durée comprise entre 0,5 et 10 heure(s), de préférence entre 1 et 4 heure(s).

Dans le cas où le catalyseur contient plusieurs métaux, ces derniers peuvent être introduits soit tous de la même façon soit par des techniques différentes, avant ou après mise en forme et dans n'importe quel ordre. Dans le cas où la technique utilisée est celle de l'échange ionique, plusieurs échanges successifs peuvent être nécessaires pour introduire les quantités requises de métaux.

La mise en forme du catalyseur selon l'invention est généralement telle que le catalyseur est sous forme de pastilles, d'agrégats, d'extrudés ou de billes, en vue de son utilisation, de préférence sous forme d'extrudés ou de billes.

Par exemple, une des méthodes préférées de préparation du catalyseur selon l'invention consiste à malaxer la zéolithe dans un gel humide de matrice (obtenu généralement par mélange d'au moins un acide et d'une poudre de matrice), par exemple d'alumine, pendant une durée nécessaire pour l'obtention d'une bonne homogénéité de la pâte ainsi obtenue, soit par exemple pendant une dizaine de minutes, puis à passer ladite pâte à travers une filière pour former des extrudés, par exemple de diamètre compris entre 0,4 et 4 mm. Puis, après séchage pendant quelques heures à 100 °C en étuve et après calcination, par exemple pendant 2 heures à 400 °C, l'élément, le rhénium, peut être déposé, par exemple par imprégnation à sec d'une solution de perrhénate d'ammonium, ledit dépôt étant suivi d'une calcination finale, par exemple pendant 2 heures à 400 °C. De manière préférée, le catalyseur obtenu est caractérisé par un coefficient de répartition macroscopique du métal, obtenu à partir de son profil déterminé par microsonde de Castaing, défini comme le rapport des concentrations dudit métal au coeur du grain par rapport au bord de ce même grain, compris de préférence entre 0,7 et 1,3 bornes incluses. De plus, de manière préférée, le catalyseur selon la présente invention, sous forme de billes ou d'extrudés, présente une valeur de l'écrasement en lit, déterminée selon la méthode Shell (SMS 1471-74) supérieure à 0,7 MPa.

La préparation du catalyseur se termine généralement par une calcination, dite calcination finale, habituellement à une température comprise entre 250 et 600 °C, de préférence précédée d'un séchage, par exemple à l'étuve, à une température généralement comprise entre la température ambiante et 250 °C, de préférence entre 40 et 200 °C. Ladite étape de séchage est de préférence menée pendant la montée en température nécessaire pour effectuer ladite calcination.

On peut procéder ensuite à une réduction sous hydrogène, généralement à une température comprise entre 300 et 600°C, de préférence entre 350 et 550°C, et pour une durée comprise entre 1 et 10 heure(s), de préférence entre 2 et 5 heures. Une telle réduction peut avoir lieu ex situ ou in situ, par rapport au lieu d'utilisation dudit catalyseur dans une réaction donnée.

Le catalyseur de la présente invention peut éventuellement contenir du soufre. Dans ce cas, le soufre est introduit sur le catalyseur mis en forme, calciné, contenant le ou les élément(s) cité(s) précédemment, soit *in situ* avant la réaction catalytique, soit *ex situ.* La sulfuration s'effectue en utilisant tout agent sulfurant bien connu de l'homme de métier, tel que par exemple le disulfure de diméthyle ou le sulfure d'hydrogène. La sulfuration éventuelle intervient après la réduction. Dans le cas d'une sulfuration *in situ,* la réduction, si le catalyseur n'a pas été préalablement réduit, intervient avant la sulfuration. Dans le cas d'une sulfuration *ex situ,* on effectue la réduction puis la sulfuration.

Le catalyseur contenant la zéolithe selon l'invention, et en particulier la NU-87, est utilisé pour la conversion d'hydrocarbures.

L'invention concerne en particulier l'utilisation dudit catalyseur en transalkylation d'hydrocarbures alkylaromatiques, et de préférence la transalkylation du toluène et d'hydrocarbures alkylaromatiques généralement en $C_9^+$ (c'est à dire à au moins 9 atomes de carbone par molécule), avec des mélanges toluène - $AC_9^+$ (où $AC_9^+$ désigne les hydrocarbures alkylaromatiques comportant au moins 9 atomes de carbone par molécule) pouvant contenir de 0 à 100% de $AC_9^+$ par rapport au mélange total. Ledit catalyseur se révèle très efficace pour ladite utilisation, car il se révèle être particulièrement actif, sélectif et stable, même en présence de charges à traiter contenant une grande quantité d'aromatiques lourds $AC_9^+$, ces aromatiques lourds pouvant contenir une grande proportion d'$AC_{10}^+$. Ainsi, des charges $AC_9^+$ contenant au moins 5 % et jusqu'à 25 % poids, et même davantage d'$AC_{10}^+$ peuvent être valorisées. A titre d'exemples, on peut citer de manière non exhaustive, les diméthyléthylbenzènes, les diéthylbenzènes, les propyléthylbenzènes... L'utilisation de ce catalyseur en transalkylation d'alkylaromatiques lourds est donc particulièrement intéressante.

Les conditions opératoires pour ladite utilisation sont généralement les suivantes : une température comprise entre 250 et 650°C et de préférence entre 350 et 550°C ; une pression comprise entre 1 et 6 MPa et de préférence entre 2 et 4,5 MPa ; une vitesse spatiale d'alimentation, exprimée en kilogramme de charge introduite par kilogramme de catalyseur et par heure, comprise entre 0,1 et 10 h$^{-1}$ et de préférence entre 0,5 et 4 h$^{-1}$ ; un rapport molaire hydrogène sur hydrocarbures compris entre 2 et 20 et de préférence entre 3 et 12 mol/mol.

## EXEMPLES

**Exemple 1** *: Préparation d'un catalyseur à base de mordénite et de rhénium, non conforme à l'invention.*

La matière première utilisée est une zéolithe mordénite, qui possède un rapport atomique Si/Al global égal à 7,6, et une teneur pondérale en sodium par rapport au poids en zéolithe mordénite sèche d'environ 3,8 %.

Cette zéolithe mordénite subit une attaque acide, à l'aide d'une solution d'acide nitrique 8N, à environ 100°C, pendant 4 heures, de manière à extraire partiellement les atomes d'aluminium présents dans la charpente zéolithique de la mordénite. La zéolithe mordénite ainsi désaluminée est ensuite soumise à un échange ionique dans une solution de $NH_4NO_3$ 10N à environ 100°C pendant 4 heures de manière à retirer le sodium résiduel.

A l'issue de ces traitements, la zéolithe mordénite sous forme H a un rapport Si/Al atomique global égal à 47,9, et une teneur pondérale en sodium par rapport au poids de zéolithe mordénite sèche de 48 ppm.

Cette zéolithe est ensuite mise en forme par extrusion avec un gel d'alumine de manière à obtenir, après séchage et calcination sous air sec, un support S1 qui contient en poids 80% de zéolithe mordénite forme H et 20 % d'alumine.

Le support S1 est ensuite imprégné à l'aide d'une solution aqueuse d'acide perrhénique, de manière à déposer 0,3 % poids de rhénium sur le solide. Le solide humide est ensuite séché à 120°C pendant 12 heures et calciné sous un débit d'air sec à la température de 500°C pendant une heure. Le catalyseur C1 ainsi obtenu contient en poids 79,7 % de mordénite, 20,0 % d'alumine et 0,29 % de Rhénium.

**Exemple 2** *:* **Préparation d'un catalyseur à base de NU-87 et de nickel, non conforme à l'invention.**

La matière première utilisée est une zéolithe NU-87, qui possède un rapport atomique Si/Al global égal à 17,2, une teneur pondérale en sodium correspondant à un rapport atomique Na/Al égal à 0,144. Cette zéolithe Nu-87 a été synthétisée d'après la demande de brevet européen EP-A-0.377.291 ou le brevet EP-B-0.378.916.

Cette zéolithe NU-87 subit, tout d'abord, une calcination dite sèche à 550°C sous flux d'air et d'azote durant 6 heures. Puis, le solide obtenu est soumis à un échange ionique dans une solution de $NH_4NO_3$ 10N, à environ 100°C pendant 4 heures. La zéolithe NU-87 est alors soumise à un traitement par une solution d'acide nitrique 7N, à environ 100°C, pendant 5 heures. Le volume V de la solution d'acide nitrique engagé (en ml) est égal à 10 fois le poids P de zéolithe NU-87 sèche (V/P=10). Ce traitement par une solution d'acide nitrique 7N est réalisé une seconde fois dans les mêmes conditions opératoires.

A l'issue de ces traitements, la zéolithe obtenue se trouve sous sa forme H et possède un rapport Si/Al atomique global égal à 34,6, et un rapport Na/Al égal à 0,007.

La zéolithe H-NU-87 est ensuite mise en forme par extrusion avec un gel d'alumine de manière à obtenir, après séchage et calcination sous air sec, le support S2 qui contient en poids 70 % de zéolithe H-NU-87 et 30 % d'alumine.

Ce support S2 est soumis à une imprégnation à sec par une solution de nitrate de nickel de manière à déposer 0,6 % poids de nickel dans le catalyseur. Le solide humide est ensuite séché à 120°C pendant 12 heures et calciné sous un débit d'air sec à la température de 500°C pendant une heure. Le catalyseur C2 obtenu contient en poids, 69,6 % de NU-87 forme hydrogène, 29,8 % d'alumine et 0,58 % de nickel.

**Exemple 3 : Préparation d'un catalyseur à base de NU-87 et de rhénium, non conforme à l'invention.**

Le support S2 est imprégné à l'aide d'une solution aqueuse de perrhénate d'ammonium de manière à déposer 0,3 % poids de rhénium sur le solide. Le solide humide est ensuite séché à 120°C pendant 12 heures et calciné sous un débit d'air sec à la température de 500°C pendant une heure. Le catalyseur C3 ainsi obtenu contient en poids 69,8 % de NU-87 forme hydrogène, 29,9 % d'alumine et 0,31 % de rhénium.

**Exemple 4 : Comparaison des performances catalytiques des catalyseurs C1 et C2 non conformes à l'invention et du catalyseur C3 non conforme à l'invention.**

Les catalyseurs sont préalablement réduits sous hydrogène à 450°C pendant 2 heures.

Le catalyseur C2 a ensuite été traité avec une charge contenant du disulfure de diméthyle (DMDS), avec une concentration telle que le rapport atomique soufre/métal soit de 1,5. Ce traitement est effectué pendant 3 heures à 400°C, en maintenant un rapport hydrogène/hydrocarbure égal à 4.

Les tests catalytiques ont été réalisés dans les conditions opératoires suivantes :

- température : 400°C
- pression totale : 30 bg
- H2/HC : 5 mol/mol.

Deux types de charges ont été utilisés :

- Une charge, notée A1, constituée essentiellement d'aromatiques à 9 atomes de carbone, contenant 4,3 % poids d'aromatiques à 10 atomes de carbone ;
- Une charge AC9+, notée A2, contenant 17,0 % poids d'aromatiques à 10 atomes de carbone.

1/ Comparaison des catalyseurs à base de NU-87 : les deux catalyseurs à base de NU-87, l'un contenant du nickel (C2 non conforme) et l'autre du rhénium (C3 non conforme) ont d'abord été comparés à iso-conditions avec une charge contenant 50 % de toluène et 50 % de charge A1. Les résultats sont présentés dans le tableau 1 :

Tableau 1

|  | C2 (non conforme) | C3 (non conforme) |
|---|---|---|
| Conversion globale (%) | 51,9 | 52,8 |
| Rendements (%poids) |  |  |
| Légers ($C_1$-$C_4$) | 3,4 | 9,8 |
| Benzène+xylènes | 41,8 | 42,8 |
| Ethylbenzène | 2,0 | 0,4 |
| Lourds | 5,1 | 1,9 |
| Ethyltoluène | 3,6 | 0,6 |
| Diméthylethylbenzène | 1,6 | 0,2 |

Le catalyseur C3 à base de rhénium présente des performances nettement améliorées par rapport au catalyseur à base de nickel (art antérieur). En effet, il est particulièrement actif avec une conversion globale plus élevée pour une teneur en métal diminuée de moitié. De plus, le rendement benzène+xylènes augmente et le faible rendement en éthylbenzène permet de faciliter la séparation du para-xylène. Enfin, le rendement en légers augmente, au détriment du rendement en lourds ce qui est favorable à la stabilité du catalyseur. Cette augmentation de la fraction légère $C_1$-$C_4$ est essentiellement due aux propriétés désalkylantes de ce nouveau catalyseur. En effet, on peut constater à l'aide des résultats présentés dans le tableau 1 que le catalyseur C3, non conforme à l'invention, désalkyle l'éthylbenzène conduisant donc à la formation de molécules en C2. L'analyse détaillée des composés aromatiques à 8, 9 et 10 atomes de carbone présents dans la charge et possédant au moins un groupement alkyl à 2 atomes de carbone ou plus montre une forte désalkylation de tous ces composés avec le catalyseur C3, ce qui conduit au rendement élevé en légers.

Le catalyseur C3, non conforme à l'invention, présente également une stabilité améliorée par rapport au catalyseur C2 non conforme. Dans le cas du catalyseur C2 à base de nickel, sous charge 50 % Toluène / 50 % A1, la conversion globale chute de 51,9 à 46,5 % en 260 heures ce qui correspond à une désactivation de 3,8 % en 100 heures. Dans le cas du catalyseur C3 à base de rhénium, on atteint une désactivation de 3,6 % en 100 heures pour une charge beaucoup plus lourde (20 % T / 80 % A2) avec une chute de conversion globale de 53,5 à 51,6 %. Or, il est bien connu de l'homme de l'art que la stabilité chute lorsque le poids moléculaire moyen de la charge augmente. Le catalyseur C3 présente donc de manière surprenante une stabilité nettement améliorée. Ceci montre bien que le catalyseur à base de rhénium est beaucoup plus stable que le catalyseur à base de nickel.

2/ Comparaison des catalyseurs contenant du Rhénium : les deux catalyseurs contenant du rhénium, l'un à base de zéolithe MOR (C1 non conforme) et l'autre à base de zéolithe NU-87 (C3 non conforme) ont été comparés à iso-conversion avec une charge contenant 20 % de toluène et 80 % de charge A2. Les résultats sont présentés dans le tableau 2 :

Tableau 2

| Charge | 20 % Toluène / 80 % A2 | |
|---|---|---|
| Catalyseurs | C1 (non conforme) | C3 (non conforme) |
| Conv. globale (%) | 53,0 | 52,8 |
| Rendements (%poids) |  |  |
| Légers C1-C4 | 12,5 | 11,8 |
| C5+ | 0,8 | 0,3 |
| Benzène | 5,8 | 5,5 |
| Xylènes | 33,3 | 34,4 |
| Ethylbenzène | 0,7 | 0,5 |
| Lourds | 0,8 | 1,1 |
| Ethyltoluène | 1,7 | 1,1 |
| Diméthyléthylbenzène | 0,9 | 0,7 |

Ces résultats permettent de montrer la meilleure sélectivité du catalyseur à base de NU-87. En effet, le rendement en xylènes est augmenté tandis que le rendement en $C_5^+$, produits secondaires non désirés de la réaction, diminue fortement. Certains des composés de cette fraction $C_5^+$ pénalisent la pureté du benzène produit. La pureté du benzène distillé peut être estimée (selon le brevet WO 98/56741) à l'aide de la formule suivante :

$$\text{Pureté benzène distillé} = 100 * Bz / (Bz + a + b + c + d)$$

Avec

a = 0,1 * Paraffines en $C_6$
b = 0,7 * Methylcyclopentane
c = Cyclohexane
d = Naphtènes en $C_7$.

Dans le cas du catalyseur C1 non conforme, on arrive à une pureté de benzène estimée de 98,17 % tandis que dans le cas du catalyseur C3, cette pureté estimée est de 99,69 %. Cette augmentation très nette de la pureté est un avantage supplémentaire lors de l'utilisation du catalyseur selon l'invention.

De plus, cette fraction $C_5^+$ contient essentiellement des paraffines à 5 et 6 atomes de carbone qui proviennent des cycles aromatiques (ouverture et craquage). Il y a donc perte de noyaux aromatiques accrue, et par suite perte en produits recherchés dans le cas du catalyseur C1 non conforme à l'invention.

L'analyse détaillée des composés aromatiques à 9 et 10 atomes de carbone contenant des groupements éthyles présentée dans le tableau 2 montre que les propriétés désalkylantes du catalyseur à base de NU-87 sont supérieures à celles du catalyseur non conforme à base de mordénite. Par exemple, la conversion de l'éthyltoluène présent à 24,25 % poids dans la charge est de 95,4 % avec C3 contre seulement 92,9 % avec C1. On constate cependant que le catalyseur C1 conduit à un rendement supérieur en fraction légère $C_1$-$C_4$. En fait, deux types de réactions produisent ces composés légers : la désalkylation des alkylaromatiques dont les groupements alkyles contiennent deux atomes de carbone ou plus et les réactions d'ouverture de cycles suivies de craquage. Le catalyseur à base mordénite conduit donc à plus de craquage et moins de désalkylation que le catalyseur à base de NU-87 qui est par conséquent plus performant pour la réaction de désalkylation qui permet de valoriser les alkylaromatiques lourds comportant des groupements éthyles, propyles...en composés (polyméthyl)benzènes valorisables en xylènes.

L'intérêt du catalyseur selon l'invention et de son utilisation en transalkylation en présence de fortes teneurs en AC9 et de fortes teneurs en AC9 et AC10 est qu'il est plus actif dans la conversion des molécules lourdes par désalkylation sélective et qu'il conduit à un rendement amélioré en xylènes et conduit à du benzène de pureté améliorée. Cette conversion des charges particulièrement lourdes est réalisée tout en conservant une très bonne stabilité du catalyseur.

## Revendications

1. Catalyseur comprenant au moins un liant, au moins une zéolithe de type structural NES, présente au moins en partie sous forme acide et comprenant du silicium et au moins un élément T choisi dans le groupe formé par l'aluminium, le fer, le gallium, le bore de l'élément T ayant été extrait de ladite tel que le rapport Si/T atomique global sont supérieur à 20, ledit catalyseur comprenant en outre au moins du rhénium et au moins un métal choisi dans le groupe formé par les métaux des groupes IIIA et IVA de la Classification périodique des Eléments.

2. Catalyseur selon la revendication 1 dans lequel le rhénium est déposé sur le mélange zéolithe-matrice.

3. Catalyseur selon la revendication 1 ou la revendication 2 tel que le rapport Si/T atomique global de la zéolithe de type structural NES est compris entre 25 et 350.

4. Catalyseur selon l'une des revendications 1 à 3 tel que la zéolithe de type structural NES est la zéolithe NU-87.

5. Catalyseur selon l'une des revendications 1 à 4 tel que la teneur en sodium est inférieure à 0,1% poids par rapport au poids total de la zéolithe de type structural NES.

6. Catalyseur selon l'une des revendications 1 à 5 que le métal choisi dans le groupe formé par les métaux des groupes IIIA et IVA est choisi parmi l'indium et l'étain.

**7.** Catalyseur selon l'une des revendications 1 à 6 comprenant du soufre.

**8.** Catalyseur selon l'une des revendications 1 à 7 comprenant en poids par rapport au poids total de catalyseur de 30 à 90% de zéolithe de type structural NES, de 0,01 à 5% poids de rhénium de 0,01 à 5% poids d'au moins un métal choisi dans le groupe formé par les métaux des groupes IIIA et IVA de la Classification périodique des Eléments et au moins une matrice en complément à 100%.

**9.** Catalyseur selon l'une des revendications 1 à 8 tel que le rhénium présente un coefficient de répartition macroscopique compris entre 0,7 et 1,3.

**10.** Catalyseur selon l'une des revendications 1 à 9 **caractérisé en ce qu'**il présente une valeur de l'écrasement en lit supérieure à 0,7 MPa.

**11.** Préparation d'un catalyseur selon l'une des revendications 1 à 10 comprenant l'extraction de l'élément T d'une zéolithe NES brute de synthèse contenant du structurant organique par la méthode d'attaque acide directe.

**12.** Préparation d'un catalyseur selon l'une des revendications 1 à 10 comprenant l'extraction de l'élément d'une zéolithe NES brute de synthèse contenant du structurant organique par la méthode de traitement thermique et d'attaque acide.

**13.** Préparation selon la revendication 12 telle que le traitement thermique est effectué en présence de vapeur d'eau.

**14.** Préparation selon l'une des revendications 11 à 13 comprenant le mélange de la matrice et de la zéolithe de type structural NES, la mise en forme du mélange et l'introduction d'au moins du rhénium et d'au moins un métal choisi dans le groupe formé par les métaux des groupes IIIA et IVA de la Classification périodique des Eléments.

**15.** Utilisation du catalyseur selon l'une des revendications 1 à 10 ou préparé selon l'une des revendications 11 à 14 dans un procédé de transalkylation d'une charge d'hydrocarbures alkylaromatiques tels que le toluène et les alkylaromatiques à au moins 9 atomes de carbone.

**16.** Utilisation selon la revendication 15 pour le traitement de charges aromatiques contenant au moins 5 % poids d'aromatiques ayant au moins 10 atomes de carbone.

**Claims**

**1.** A catalyst comprising at least one binder, at least one zeolite with structure type NES at least partially in its acid form and comprising silicon and at least one element T selected from the group formed by aluminium, iron, gallium and boron, some element T having been extracted from said zeolite so that the overall Si/T atomic ratio is more than 20, said catalyst further comprising at least rhenium and at least one metal selected from the group formed by metals from groups IIIA and IVA of the periodic table.

**2.** A catalyst according to claim 1, in which rhenium is deposited on the mixture zeolite-matrix.

**3.** A catalyst according to claim 1 or claim 2, in which the overall Si/T atomic ratio of the zeolite with structure type NES is in the range 25 to 350.

**4.** A catalyst according to any one of claims 1 to 3, in which the zeolite with structure type NES is NU-87 zeolite.

**5.** A catalyst according to any one of claims 1 to 4, in which the sodium content is less than 0.1% by weight with respect to the total weight of zeolite with structure type NES.

**6.** A catalyst according to any one of claims 1 to 5 in which the metal selected from the group formed by metals from groups IIIA and IVA of the periodic table is selected from indium and tin.

**7.** A catalyst according to any one of claims 1 to 6 comprising sulphur.

**8.** A catalyst according to any one of claims 1 to 7 comprising, by weight with respect to the total catalyst weight, 30% to 90% of the zeolite with structure type NES and 0.01% to 5% by weight of rhenium, 0.01% to 5% by weight of at least one metal selected from the group formed by metals from groups IIIA and IVA of the periodic table and at least one matrix making up the complement to 100%.

9. A catalyst according to any one of claims 1 to 8, in which the macroscopic distribution coefficient of rhenium is in the range 0.7 to 1.3.

10. A catalyst according to any one of claims 1 to 9, **characterized in that** the bed crush strength is more than 0.7 MPa.

11. Preparation of a catalyst according to any one of claims 1 to 10, comprising the extraction of element T from an as synthesised NES zeolite containing an organic template using the direct acid attack method.

12. Preparation of a catalyst according to any one of claims 1 to 10, comprising the extraction of element T from an as synthesised NES zeolite containing an organic template using the heat treatment and acid attack method.

13. Preparation according to claim 12 in which the heat treatment is carried out in the presence of steam.

14. Preparation according to any one of claims 11 to 13, comprising mixing the matrix and the zeolite with structure type NES, forming the mixture and introducing at least rhenium and at least one metal selected from the group formed by metals from groups IIIA and IVA of the periodic table.

15. Use of a catalyst according to any one of claims 1 to 10 or prepared according to any one of claims 11 to 14 in a process for transalkylating an alkylaromatic hydrocarbon feed such as toluene and alkylaromatic compounds containing at least 9 carbon atoms.

16. Use according to claim 15, for the treatment of aromatic feeds containing at least 5% by weight of aromatic compounds containing at least 10 carbon atoms.

**Patentansprüche**

1. Katalysator, umfassend mindestens ein Bindemittel, mindestens einen Zeolith des NES-Strukturtyps, der zumindest teilweise in saurer Form vorhanden ist, und umfassend ein Silizium und mindestens ein Element T, das in der Gruppe, die von Aluminium, Eisen, Gallium, Bor gebildet ist, ausgewählt wird, wobei das Element T, das aus dem Zeolith extrahiert wurde, derart ausgewählt wird, dass das Gesamtatomverhältnis Si/T größer als 20 ist, wobei der Katalysator ferner mindesten Rhenium und mindestens ein Metall umfasst, das in der Gruppe, die von den Metallen der Gruppen IIIA und IVA des Periodensystems der Elemente gebildet ist, ausgewählt wird.

2. Katalysator nach Anspruch 1, bei dem das Rhenium auf das Zeolith-Matrix-Gemisch aufgebracht wird.

3. Katalysator nach Anspruch 1 oder Anspruch 2, bei dem das Gesamtatomverhältnis Si/T des Zeoliths des NES-Strukturtyps zwischen 25 und 350 beträgt.

4. Katalysator nach einem der Ansprüche 1 bis 3, bei dem der Zeolith des NES-Strukturtyps der Zeolith NU-87 ist.

5. Katalysator nach einem der Ansprüche 1 bis 4, bei dem der Natriumgehalt geringer als 0,1 Gew.-% bezogen auf das Gesamtgewicht des Zeoliths des NES-Strukturtyps ist.

6. Katalysator nach einem der Ansprüche 1 bis 5, bei dem das Metall, das in der Gruppe, die von den Metallen der Gruppen IIIA und IVA gebildet ist, ausgewählt wird, unter Indium und Zinn ausgewählt wird.

7. Katalysator nach einem der Ansprüche 1 bis 6, umfassend Schwefel.

8. Katalysator nach einem der Ansprüche 1 bis 7, umfassend in Gewicht bezogen auf das Gesamtgewicht des Katalysators 30 bis 90 % eines Zeoliths des NES-Strukturtyps, 0,01 bis 0,5 Gew.-% Rhenium, 0,01 bis 5 Gew.-% von mindestens einem Metall, das in der Gruppe, die von den Metallen der Gruppen IIIA und IVA des Periodensystems der Elemente gebildet ist, ausgewählt wird, und mindestens eine Matrix zur Ergänzung auf 100 %.

9. Katalysator nach einem der Ansprüche 1 bis 8, bei dem das Rhenium einen makroskopischen Verteilungskoeffizienten zwischen 0,7 und 1,3 aufweist.

10. Katalysator nach einem der Ansprüche 1 bis 9, **dadurch gekennzeichnet, dass** er einen Wert der Druckverformung im Bett über 0,7 MPa aufweist.

11. Herstellung eines Katalysators nach einem der Ansprüche 1 bis 10, umfassend die Extraktion des Elements T eines rohen Synthese-NES-Zeoliths, umfassend ein organisches Strukturmittel, durch die Methode des direkten Säureangriffs.

12. Herstellung eines Katalysators nach einem der Ansprüche 1 bis 10, umfassend die Extraktion des Elements T eines rohren Synthese-NES-Zeoliths, umfassend ein organisches Strukturmittel, durch die Methode der Wärmebehandlung und des Säureangriffs.

13. Herstellung nach Anspruch 12, bei der die Wärmebehandlung im Beisein von Wasserdampf erfolgt.

14. Herstellung nach einem der Ansprüche 11 bis 13, umfassend das Mischen der Matrix und des Zeoliths des NES-Strukturtyps, die Formung des Gemisches und die Einführung von mindestens Rhenium und mindestens einem Metall, das in der Gruppe, die von den Metallen der Gruppen IIIA und IVA des Periodensystems der Elemente gebildet ist, ausgewählt wird.

15. Verwendung des Katalysators nach einem der Ansprüche 1 bis 10 oder hergestellt nach einem der Ansprüche 11 bis 14 in einem Transalkylierungsverfahren von alkylaromatischen Kohlenwasserstoffen, wie beispielsweise Toluen und die alkylaromatischen Stoffe mit mindestens 9 Kohlenstoffatomen.

16. Verwendung nach Anspruch 15 für die Behandlung von aromatischen Füllstoffen, die mindestens 5 Gew.-% von aromatischen Stoffen enthalten, die mindestens 10 Kohlenstoffatome umfassen.